# EUROPEAN PATENT APPLICATION

(11) **EP 4 837 614 A1**
(43) Date of publication of application: **11.11.2026**
(21) Application number: 25751306.9
(22) Date of filing: 23.01.2025
(51) Int. Cl.: A61M 25/10

(54) **DILATION BALLOON AND DILATION BALLOON ASSEMBLY**

(30) Priority: 06.02.2024 CN 202410171884
(71) Applicant: BEIJING YILEI WANJUN TECHNOLOGY DEVELOPMENT CO., LTD., Beijing 101115 (CN)
(72) Inventor: LI, Lei, Beijing 101115 (CN); LI, Huajun, Beijing 101115 (CN)
(74) Representative: Icosa
(86) International application number: PCT/CN2025/074376
(87) International publication number: WO 2025/167704

(57) **Abstract**

A dilation balloon and a dilation balloon assembly. The dilation balloon includes a balloon body, including an inner layer and an outer layer. The balloon body is configured to expand or retract as a medium is filled into or discharged from the balloon body. The outer layer is configured to abut against a vascular inner wall and dilate a blood vessel upon expansion of the balloon body. The balloon body is configured to move within the blood vessel upon retraction of the balloon body. The inner layer is a non-compliant layer, and the outer layer is a compliant layer.

## Description

### CROSS REFERENCE TO RELATED APPLICATIONS

The present disclosure claims priority to Chinese Patent Application No. 202410171884.0, filed on February 6, 2024, entitled "DILATION BALLOON AND DILATION BALLOON ASSEMBLY," the entire contents of which are incorporated herein by reference.

### TECHNICAL FIELD

The present disclosure relates to the technical field of medical devices and specifically relates to a dilation balloon and a dilation balloon assembly.

### BACKGROUND OF THE INVENTION

In the treatment of lesions such as vascular stenosis, balloon catheter dilation is widely used. In the related art, a non-compliant balloon is generally used as a dilation balloon to endow the dilation balloon with a higher radial support force. The non-compliant balloon usually needs to be pre-crimped to fold the balloon, and after the folded balloon is delivered to the lesion site, the balloon is pressurized to expand, so as to dilate the stenotic lesion site. However, during expansion of the above-described non-compliant balloon, since the balloon in a folded state is generally in a multi-layer folded state, each folded layer unfolds as the balloon continuously expands under pressurization and causes a rotational effect, which tends to cause vascular dissection.

### SUMMARY OF THE INVENTION

The objective of the present disclosure is to provide a dilation balloon and a dilation balloon assembly. The dilation balloon can achieve uniform dilation of a vascular inner wall, avoid tearing at local positions of the vascular inner wall, and effectively reduce the risk of vascular dissection.

In order to achieve the above object, a dilation balloon is provided by a first aspect of the present disclosure. The dilation balloon includes a balloon body, and the balloon body includes an inner layer and an outer layer. The balloon body is configured to expand or retract as a medium is filled into or discharged from the balloon body. The outer layer is configured to abut against a vascular inner wall and dilate a blood vessel upon expansion of the balloon body. The balloon body is configured to move within the blood vessel upon retraction of the balloon body. The inner layer is a non-compliant layer, and the outer layer is a compliant layer.

In some examples, the balloon body further includes a transition layer disposed between the inner layer and the outer layer.

In some examples, the transition layer is filled with lubricating fluid.

In some examples, the outer layer includes a first compliant layer on an inner side and a second compliant layer on an outer side. A rigid protrusion is disposed between the first compliant layer and the second compliant layer. A wall surface of the second compliant layer undergoes expansion and thinning upon expansion of the balloon body, and the rigid protrusion is configured to push a portion of the wall surface of the second compliant layer outward such that a bump for abutting against the vascular inner wall is formed on the second compliant layer.

In some examples, a plurality of the rigid protrusions are provided and arranged in an array.

In some examples, a spacing between any two adjacent rigid protrusions among the plurality of rigid protrusions is equal.

In some examples, the rigid protrusion is integrally formed with the first compliant layer from a polymeric material.

In some examples, an end of the rigid protrusion toward the second compliant layer is arcuate.

In some examples, the dilation balloon further includes a connecting catheter, and the balloon body is disposed around one end of the connecting catheter, such that the medium is filled into or discharged from the balloon body through the connecting catheter.

A dilation balloon assembly is further provided by a second aspect of the present disclosure. The dilation balloon assembly includes a syringe and a dilation balloon as described above, and the syringe is configured to fill a medium into or discharge a medium from the balloon body.

With the above technical solution, i.e., the dilation balloon provided by the present disclosure, the dilation balloon is configured to expand or retract upon filling of the medium into or discharge of the medium from the dilation balloon. Accordingly, a dilation treatment can be achieved at a stenotic lesion site within a blood vessel, for example, in a vascular dilation treatment. Meanwhile, the inner layer and the outer layer of the balloon body are configured as the non-compliant layer and the compliant layer, respectively, so that when the balloon body expands and abuts against the vascular inner wall, the compliant layer can fit against the vascular inner wall. As such, the compliant layer is capable of uniform expansion without rotation during the expansion of the balloon body, which facilitates uniform stress distribution on the vascular inner wall, avoids tearing at local positions of the vascular inner wall, and effectively reduces the risk of vascular dissection. Furthermore, the inner layer is configured as the non-compliant layer, so that a greater radial support force can be provided by the non-compliant layer when the balloon body abuts against the vascular inner wall, thereby improving the dilation treatment effect of the dilation balloon on the stenotic site within the blood vessel.

Other features and advantages of the present disclosure will be described in detail in the following DETAILED DESCRIPTION OF THE INVENTION.

### BRIEF DESCRIPTION OF DRAWINGS

The accompanying drawings are provided to facilitate further understanding of the present disclosure and constitute a part of the specification, and together with the following detailed description serve to explain the present disclosure, but do not constitute a limitation of the present disclosure. In the drawings:
FIG. 1 is a schematic structural view of a dilation balloon provided in a related art disposed at a vascular inner wall;
FIG. 2 is a sectional view of position A-A in FIG. 1;
FIG. 3 is a schematic structural view of a dilation balloon provided in an example of the present disclosure in a retracted state;
FIG. 4 is an enlarged partial schematic view of position B in Figure 3;
FIG. 5 is a schematic structural view of a dilation balloon provided in an example of the present disclosure in an expanded state;
FIG. 6 is an enlarged partial schematic view of position C in Figure 5;
FIG. 7 is a schematic structural view of a dilation balloon provided in an example of the present disclosure disposed at a vascular inner wall .

### Description of Reference Numerals

1 - balloon body; 110 - inner layer; 120 - outer layer; 121 - first compliant layer; 122 - second compliant layer; 123 - bump; 130 - transition layer; 2-lubricating fluid; 3 - rigid protrusion; 4 - vascular inner wall; 5 - connecting catheter; 510 - first inlet; 6 - non-compliant balloon.

### DETAILED DESCRIPTION OF THE INVENTION

Specific embodiments of the present disclosure are described in detail below with reference to the accompanying drawings. It is to be understood that the specific embodiments described here are merely used to illustrate and interpret the present disclosure and are not used to limit the present disclosure.

In the present disclosure, unless otherwise specified, the terms "inner" and "outer" refer to the inner and outer sides relative to the contour of a component or structure itself. In addition, it is noted that terms such as "first" and "second" are adopted to distinguish one element from another without sequence or importance. Besides, in the descriptions with reference to the accompanying drawings, identical reference numerals in different accompanying drawings denote identical elements.

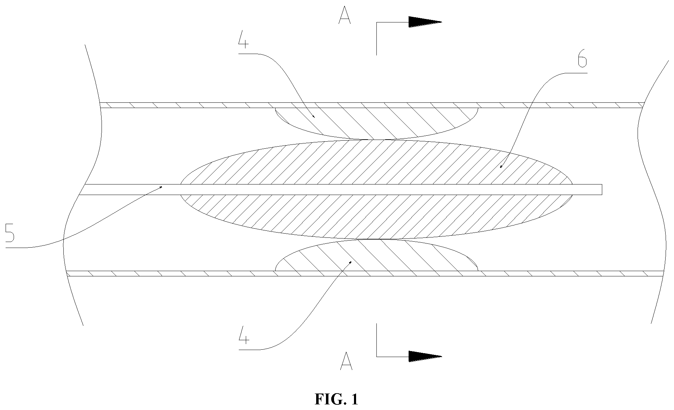

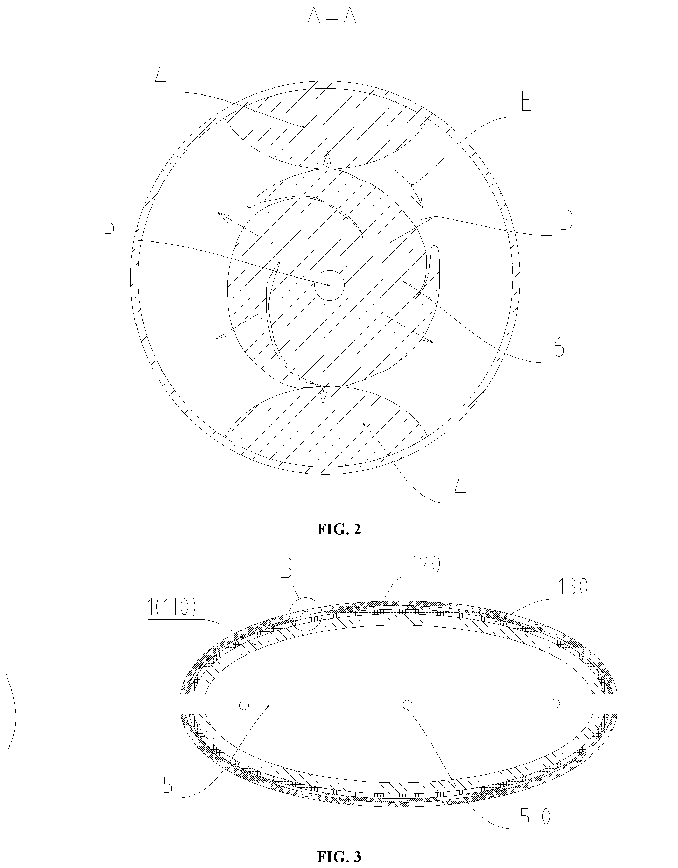

In the related art, as shown in FIG. 1 and FIG. 2, a non-compliant balloon 6 is generally used as a dilation balloon to endow the dilation balloon with a higher radial support force .The non-compliant balloon usually needs to be pre-crimped to fold the balloon, to facilitate delivery of the dilation balloon to a lesion site such as a stenosis in a blood vessel, after the folded balloon is delivered to the lesion site, the balloon is pressurized to expand outward in a direction indicated by, for example, arrow D, so as to dilate the stenotic lesion site. However, as shown in FIG. 2, during expansion of the non-compliant balloon 6, since the balloon in a folded state is generally in a multi-layer folded state, each folded layer unfolds as the balloon continuously expands by the pressurizing operation and causes rotational movement, that is, as the balloon continuously expands, a rotational effect generated upon unfolding of each layer create shear stress in a direction of, for example, arrow E, this shear stress tends to cause scraping between the portion of the non-compliant balloon 6 where it meets a vascular inner wall 4 and the vascular inner wall 4, causing tearing at local positions of the vascular the inner wall 4, which tends to cause a risk of vascular dissection.

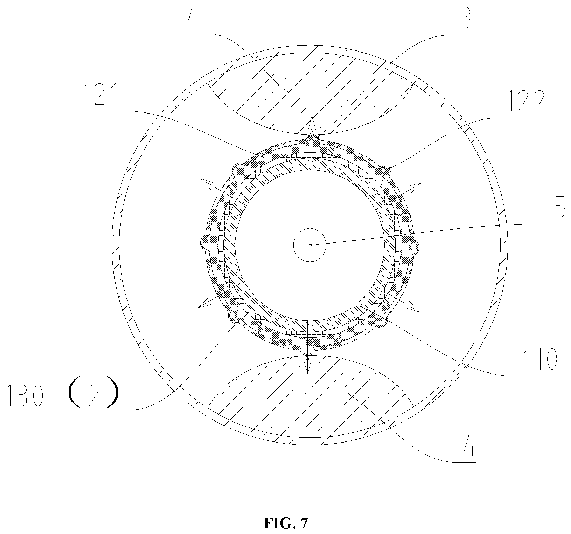

Based on this, a dilation balloon is provided according to a first aspect of the present disclosure, referring to FIGS. 3 to 7, the dilation balloon includes a balloon body 1, and the balloon body 1 includes an inner layer 110 and an outer layer 120. The balloon body 1 can expand or retract upon filling of a medium into or discharge of the medium from the balloon body 1. During expansion of the balloon body 1, the outer layer 120 can abut against the vascular inner wall 4 and dilate the blood vessel with expansion of the balloon body 1. The balloon body 1 can move within the blood vessel upon retraction of the balloon body 1. The inner layer 110 is a non-compliant layer, and the outer layer 120 is a compliant layer.

With the above technical solution, i.e., the dilation balloon provided by the present disclosure, the dilation balloon is configured to expand or retract upon filling of the medium into or discharge of the medium from the dilation balloon. Accordingly, a dilation treatment can be achieved at a stenotic lesion site within a blood vessel, for example, in a vascular dilation treatment. Meanwhile, the inner layer 110and the outer layer 120 of the balloon body 1 are configured as the non-compliant layer and the compliant layer, respectively, so that when the balloon body 1 expands and abuts against the vascular inner wall, the compliant layer can fit against the vascular inner wall 4. As such, the compliant layer is capable of uniform expansion without rotation during expansion of the balloon body 1, which facilitates uniform stress distribution of the vascular inner wall 4, avoids tearing at local positions of the vascular inner wall 4, and effectively reduces the risk of vascular dissection. Furthermore, the inner layer 110 is configured as the non-compliant layer, so that a greater radial support force can be provided by the non-compliant layer when the balloon body 1 abuts against the vascular inner wall 4, thereby improving the dilation treatment effect of the dilation balloon on the stenotic site within the blood vessel.

It is noted that, the above-mentioned compliant layer may be a polymeric material such as polyvinyl chloride (PVC), that is, it can be understood that an expansion effect of the compliant layer is similar to an expansion effect of a balloon. Thus, it is advantageous to ensure uniform expansion of the compliant layer without causing a rotation effect after the balloon body 1 expands, so that the vascular inner wall 4 is uniformly stressed, tearing at local positions of the vascular inner wall 4 is avoided, and the risk of vascular dissection is effectively reduced. Also, In view of the fact that the compliant layer is made of relatively soft material and provides low radial support force during expansion of the dilation balloon, the inner layer 110 of the balloon body 1 disposed inside the outer layer 120 is configured as the non-compliant layer in the present disclosure. For example, the non-compliant layer may be formed of polymeric materials such as polyethylene (PE), polyurethane, or Nylon (Duralyn ^{™}). Due to inherent characteristics of the non-compliant layer, which is relatively stiff with respect to the compliant layer, so that a larger radial support force can be provided, thereby the dilation treatment effect of the dilation balloon on the stenotic site within the blood vessel can be improved. Meanwhile, since the compliant layer covers the exterior of the non-compliant layer, it is also possible to avoid tearing at local positions of the vascular inner wall 4 that may occur, for example, in related art where the non-compliant layer directly contacts the vascular inner wall 4, thereby effectively reducing the risk of vascular dissection.

Clearly, it needs to be noted that the foregoing specific embodiments of the compliant and non-compliant layers are illustrative. In other embodiments, those skilled in the art may also adaptively design the compliant layer and the non-compliant layer according to practical application requirements, so long as the objectives of enabling the dilation balloon to achieve a stable dilation of the vascular inner wall 4, ensuring uniform stress on the vascular inner wall 4, avoiding tearing at local positions of the vascular inner wall 4, and reducing the risk of dissection. The present disclosure is not limited in this respect.

In some embodiments, referring to FIGS. 3 to 7, the balloon body 1 may further include a transition layer 130 disposed between the inner layer 110 and the outer layer 120. As such, stable connection between the inner layer 110 and the outer layer 120 can be achieved. Meanwhile, friction between the inner layer 110 and the outer layer 120 during expansion of the balloon body 1 can be reduced, allowing the balloon body 1 to expand uniformly, avoiding tearing at local positions of the vascular inner wall 4, and effectively reducing the risk of vascular dissection.

It needs to be noted that the inner layer 110, the outer layer 120, and the transition layer 130 can be fixedly connected to each other by, for example, adhesive bonding, and the structure is simple and reliable. Clearly, in other embodiments, the inner layer 110, the outer layer 120, and the transition layer 130 may also be integrally formed by, for example, 3D printing. The present disclosure does not limit such variations, and those skilled in the art may adaptively design according to practical application requirements, so long as stable connection among the inner layer 110, the outer layer 120, and the transition layer 130 can be achieved. The present disclosure is not limited in this respect.

Alternatively, in some embodiments, referring to FIGS. 3 to 6, the transition layer 130 may be filled with lubricating fluid 2 so as not only to further reduce the friction between the inner layer 110 and the outer layer 120, but also to enable the lubricating fluid 2 to perform a buffering function, This helps ensure uniform dilation of the vascular inner wall 4, avoids tearing at local positions of the vascular inner wall 4, and effectively reducing the risk of vascular dissection.

The foregoing lubricating fluid 2 may be a buffer solution such as a phosphate solution, a borate solution, a carbonate solution, and the like. The present disclosure does not specifically limit this. Those skilled in the art can adaptively design according to practical application requirements, so long as friction between the inner layer 110 and the outer layer 120 can be reduced, the balloon body 1 can expand uniformly, and the risk of vascular dissection can be reduced. The present disclosure is not limited in this respect.

In some embodiments, referring to FIGS. 3 to 7, the outer layer 120 may include a first compliant layer 121 on an inner side and a second compliant layer 122 on an outer side. In view of the fact that when the compliant layer of the balloon body 1 abuts against the vascular inner wall 4, direct contact between the compliant layer and the inner vessel wall 4 results in relatively low friction between the compliant layer and the vascular inner wall 4. Scraping is likely to occur, which causes tearing at local positions of the vascular inner wall 4 and readily gives rise to a risk of vascular dissection. Accordingly, the present disclosure provides a rigid protrusion 3 disposed between the first compliant layer 121 and the second compliant layer 122. In this way, as the balloon body 1 expands, a wall surface of the second compliant layer 122 continuously expands and thins, and the rigid protrusion 3 can push a portion of the wall surface of the second compliant layer 122 outward such that a bump 123 for abutting against the vascular inner wall 4 is formed on the second compliant layer 122. Thus, the frictional force between the compliant layer and the vascular inner wall 4 can be increased, the problem of scraping caused by lower frictional force which lead to tearing at local positions of the vascular inner wall 4 is avoided, and the risk of vascular dissection is effectively reduced.

In addition, when the balloon body 1 is in a retracted state, the rigid protrusion 3 can also be hidden between the first compliant layer 121 and the second compliant layer 122, facilitating delivery of the balloon body 1 of the dilation balloon to stenotic lesion site within the blood vessel. Meanwhile, the risk of scraping damage to the vascular inner wall 4 during delivery of the balloon body 1 is also avoided due to the hiding of the rigid protrusion 3 between the first compliant layer 121 and the second compliant layer 122.

Alternatively, in some embodiments, referring to FIGS. 3 to 7, a plurality of the rigid protrusions 3 can be provided and arranged in an array, so that the frictional force between the compliant layer and the vascular inner wall 4 can be further increased, avoiding the problem of scraping caused by lower frictional force which lead to tearing at local positions of the vascular inner wall 4, and effectively reducing the risk of vascular dissection. Where the plurality of rigid protrusions 3 may be arranged at intervals in, for example, a rectangular array and/or an annular array, such variations are not particularly limited in the present disclosure, and those skilled in the art can design adaptively according to practical application requirements.

In addition, in some embodiments, with reference to FIGS. 3 to 7, a spacing between any two adjacent rigid protrusions 3 among the plurality of rigid protrusions 3 may be equal, which can advantageously ensure that the vascular inner wall 4 is uniformly stressed, and effectively reducing the risk of vascular dissection.

Furthermore, in some embodiments, the rigid protrusion 3 may be integrally formed with the first compliant layer 121 from a polymer material. For example, the rigid protrusion 3 may be made of relatively stiff polymer materials such as polyethylene (PE), polyurethane, or nylon (Nylon, Duralyn ^{™}), and may be integrally formed with the first compliant layer 121 by, for example, 3D printing, which is simple and convenient to form and manufacture.

Clearly, it needs to be noted that, the manner in which the above-described rigid protrusion 3 can be made integrally with the first compliant layer 121 is illustrative. In other embodiments, the rigid protrusion 3 may also be fixedly attached to the first compliant layer 121 by means of such as laser welding or thermal fusion welding. This variation is not particularly limited by the present disclosure and can be adaptively designed by those skilled in the art according to practical application requirements. The present disclosure is not limited in this respect.

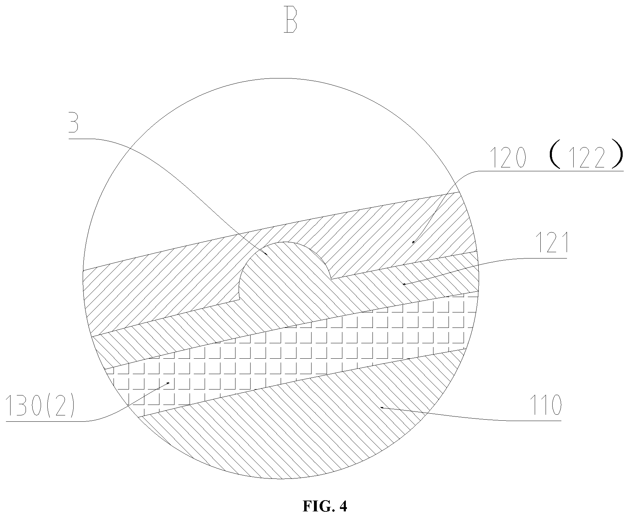

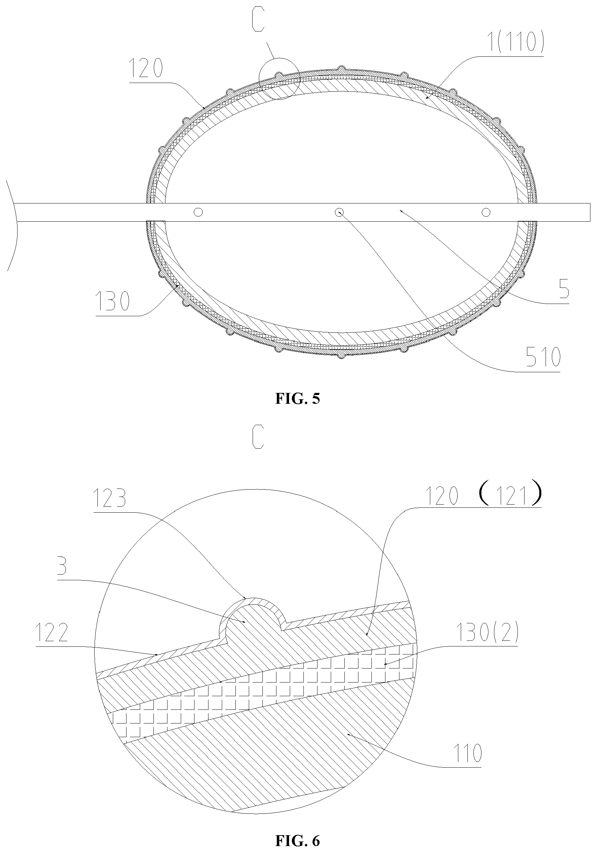

In additional, in some embodiments, referring to FIGS. 4 and 6, an end of the rigid protrusion 3 toward the second compliant layer 122 may be arcuate. As such, relatively high friction between the compliant layer and the vascular inner wall 4 can be achieved while avoiding abrasion and rupture of the vascular inner wall 4, which helps improve the dilation treatment effect of the dilation balloon on the stenotic site within the blood vessel. By way of example, it is illustratively shown in both FIG. 4 and FIG. 6 that the outer contour of the rigid protrusion 3 can be hemispherical, the structure is simple and easy to manufacture. Clearly, it needs to be explained that the specific outer contour of the above rigid protrusion 3 can also be adaptively designed by those skilled in the art according to practical application requirements. The present disclosure is not limited in this respect.

In some embodiments, referring to FIGS. 3 to 7, the dilation balloon may further include a connecting catheter 5, the balloon main body 1 is disposed around one end of the connecting catheter 5, such that the medium is filled into or discharged from the balloon main body 1 through the connecting catheter 5, which provides favorable maneuverability and simple structure. Meanwhile, it also facilitates the delivery of the dilation balloon to the stenotic lesion site within the blood vessel through the connecting catheter 5. By way of example, it is noted that, in order to deliver the dilating balloon to the stenotic lesion site within the blood vessel with higher precision, those skilled in the art may adopt well-known angiography techniques in the field to deliver the dilation balloon to the corresponding stenotic lesion site within the blood vessel. The angiography technique is a well-known technical means in the art, and the present disclosure will not be described in more detail herein. It suffices to achieve delivery of the dilation balloon to the stenotic lesion site within the blood vessel.

In addition, it needs to be noted that the above-mentioned medium may be, for example, a gas medium or a liquid medium, so that the balloon body 1 can expand after filling of the medium into the balloon body 1. The present disclosure does not specifically limit such variations, and those skilled in the art can adaptively design according to the practical application requirements.

Alternatively, in some embodiments, the balloon body 1 may be adhesively connected with the connecting catheter 5, with a simple structure and convenient mounting operation. Clearly, in other embodiments, the balloon body 1 may also be hermetically sewn to the connecting catheter 5. The present disclosure does not specifically limit such variations so as to achieve a stable connection between the connecting catheter 5 and the balloon body 1. The present disclosure is not limited in this respect.

In addition, it needs to be described that the material and the specific molding process of the above-mentioned connecting catheter 5 are not specifically limited in the present disclosure, and those skilled in the art can adaptively design according to the practical application requirements. For example, the material of the connecting catheter 5 can be nylon, polyethylene terephthalate or polyurethane, and the connecting catheter 5 may be manufactured by, for example, integral stretch blow molding or 3D printing. The present disclosure is not limited in this respect.

Furthermore, in some embodiments, referring to FIGS. 3 to 6, the connecting catheter 5 may also be provided with a first inlet 510 in communication with the interior of the balloon body 1 to facilitate filling of the medium into or discharge of the medium from the balloon body 1 through the first inlet 510 of the connecting catheter 5 after the operator delivers the dilation balloon to the stenotic lesion site within the blood vessel, thereby effecting the dilation treatment of the stenotic site in the blood vessel by the dilation balloon.

In addition, it needs to be noted that the lubricating fluid 2 in the transition layer 130 may be pre-sealed into the transition layer 130. Alternatively, the connecting catheter 5 may also be provided with a second inlet (not shown) in communication with the transition layer 130 to enable filling of the lubricating fluid 2 into or discharge of the lubricating fluid 2 from the transition layer 130 by means of the connecting catheter 5. Meanwhile, the connecting catheter 5 may further be provided with an outlet (not shown) in communication with the transition layer 130. Accordingly, gas inside the transition layer 130 can be discharged through the outlet while an operator continuously fills the lubricating fluid 2 into the transition layer 130 through the second inlet, thereby facilitating filling of the lubricating fluid 2 with favorable maneuverability.

It needs to be noted that the specific positions and opening dimensions of the second inlet and the outlet in the connecting catheter 5 are not specifically limited in the present disclosure, and those skilled in the art can adaptively design according to the practical application requirements, so long as to facilitate filling of the lubricating fluid 2 into or discharge of the lubricating fluid 2 from the transition layer 130 through the connecting catheter 5.

A dilation balloon assembly is also provided according to a second aspect of the present disclosure. The dilation balloon assembly includes a syringe (not shown) and a dilation balloon as described above, a head of the syringe is adapted to match with the connecting catheter 5, such that the syringe is configured to fill a medium into or discharge a medium from the balloon body 1. The dilation balloon assembly can be applied to scenarios for treating lesions such as vascular stenosis. The dilation balloon of the dilation balloon assembly may be positioned at a stenotic lesion site within a blood vessel, such that the dilation treatment of the stenotic lesion site within the blood vessel can be achieved by expansion of the dilation balloon. The dilation balloon assembly features favorable maneuverability and can avoid tearing at local positions of the vascular inner wall, thereby effectively reducing the risk of vascular dissection. In addition, the dilation balloon assembly possesses all the beneficial effects of the dilation balloon described above, which will not be repeated herein by the present disclosure.

In addition, it needs to be noted that the present disclosure imposes no specific limitations on the specific structure of the above-mentioned syringe and the specific connection means between the syringe and the connecting catheter 5. It suffices to enable filling of the medium into or discharge of the medium from the balloon body 1 of the dilation balloon by the syringe, and those skilled in the art can adaptively design according to the practical application requirements.

The preferred embodiments of the present disclosure are described in detail above with reference to the accompanying drawings. However, the present disclosure is not limited to the specific details of the foregoing embodiments. Within the scope of the technical concept of the present disclosure, various simple modifications may be made to the technical solution of the present disclosure, and all these simple modifications fall within the protection scope of the present disclosure.

It needs to be further noted that each specific technical feature described in the foregoing detailed embodiments may be combined in any appropriate manner without conflict. To avoid unnecessary repetition, various possible combinations will not be described separately in the present disclosure.

Furthermore, various different embodiments of the present disclosure may be combined arbitrarily without departing from the spirit of the present disclosure, and such combinations are also regarded as content disclosed by the present disclosure.

## Claims

1. A dilation balloon, comprising a balloon body (1), wherein the balloon body (1) comprises an inner layer (110) and an outer layer (120), and the balloon body (1) is configured to expand or retract as a medium is filled into or discharged from the balloon body (1), wherein the outer layer (120) is configured to abut against a vascular inner wall (4) and dilate a blood vessel upon expansion of the balloon body (1), and the balloon body (1) is configured to move within the blood vessel upon retraction of the balloon body (1), wherein the inner layer (110) is a non-compliant layer, and the outer layer (120) is a compliant layer.

2. The dilation balloon according to claim 1, wherein the balloon body (1) further comprises a transition layer (130) disposed between the inner layer (110) and the outer layer (120).

3. The dilation balloon according to claim 2, wherein the transition layer (130) is filled with lubricating fluid (2).

4. The dilation balloon according to any one of claims 1-3, wherein the outer layer (120) comprises a first compliant layer (121) on an inner side and a second compliant layer (122) on an outer side, and a rigid protrusion (3) is disposed between the first compliant layer (121) and the second compliant layer (122), wherein a wall surface of the second compliant layer (122) undergoes expansion and thinning upon expansion of the balloon body (1), and the rigid protrusion (3) is configured to push a portion of the wall surface of the second compliant layer (122) outward such that a bump (123) for abutting against the vascular inner wall (4) is formed on the second compliant layer (122) .

5. The dilation balloon according to claim 4, wherein a plurality of the rigid protrusions (3) are provided and arranged in an array.

6. The dilation balloon according to claim 5, wherein a spacing between any two adjacent rigid protrusions (3) among the plurality of rigid protrusions (3) is equal.

7. The dilation balloon according to claim 4, wherein the rigid protrusion (3) is integrally formed with the first compliant layer (121) from a polymeric material.

8. The dilation balloon according to claim 4, wherein an end of the rigid protrusion (3) toward the second compliant layer (122) is arcuate.

9. The dilation balloon according to claim 1, further comprising a connecting catheter (5), wherein the balloon body (1) is disposed around one end of the connecting catheter (5), such that the medium is filled into or discharged from the balloon body (1) through the connecting catheter (5).

10. A dilation balloon assembly, comprising a syringe and a dilation balloon according to any one of claims 1-9, wherein the syringe is configured to fill a medium into or discharge a medium from the balloon body (1).
